# NOUVEAU FASCICULE DE BREVET EUROPEEN

(11) **EP 0 812 184 B2**
(45) Date de publication et mention de la décision concernant l'opposition: **18.02.2009**
(45) Mention de la délivrance du brevet: 07.05.2003
(21) Numéro de dépôt: 96904906.3
(22) Date de dépôt: 26.02.1996
(51) Int. Cl.: A61Q 19/00, A61K 31/195, A61K 31/03, A61K 31/155

(54) **INHIBITEURS DE NO-SYNTHASE À USAGE TOPIQUE**
INHIBITOREN DER NO-SYNTHASE ZUR TOPISCHEN VERWENDUNG
NITRIC OXIDE SYNTHASE INHIBITORS FOR TOPICAL USE

(30) Priorité: 27.02.1995 FR 9502267
(43) Date de publication de la demande: 17.12.1997
(73) Titulaire: L'ORÉAL, 75008 Paris (FR)
(72) Inventeur: GIACOMONI, Paolo, F-91400 Orsay (FR)
(74) Mandataire: Bernstein, Claire Jacqueline
(86) Numéro de dépôt international: PCT/FR1996/000296
(87) Numéro de publication internationale: WO 1996/026711

(56) Documents cités:
- EP-A- 0 096 521
- EP-A- 0 249 736
- EP-A- 0 366 990
- EP-A- 0 413 528
- EP-A- 0 630 649
- WO-A-93/24126
- WO-A-95/13805
- WO-A-95/24884
- GB-A- 2 263 111
- DATABASE WPI Week 9546 Derwent Publications Ltd., London, GB; AN 95-355173 XP002003281 & JP,A,07 242 542 (NONOGAWA SHOJI KK) , 19 Septembre 1995

## Description

La présente invention concerne une utilisation d'une quantité efficace d'au moins un inhibiteur de NO-synthase pour la fabrication d'une composition pharmaceutique topique, cet inhibiteur ou la composition pharmaceutique étant destiné à diminuer l'effet irritant cutané de produits utilisés topiquement dans le domaine cosmétique ou pharmaceutique.

Elle concerne également une composition à usage topique cosmétique ou pharmaceutique comprenant une quantité efficace d'au moins un inhibiteur de NO-synthase et un procédé de traitement cosmétique mettant en oeuvre la composition cosmétique selon l'invention.

Dans le cadre de la présente invention, l'effet irritant cutané est une réponse de la peau se traduisant le plus souvent par des rougeurs, douleurs ou picotements, cette réponse étant engendrée par des produits chimiques d'origine naturelle ou synthétique appliqués de manière topique sur la peau. Cette irritation s'accompagne d'une altération de la fonction et/ou de la structure épithéliale, directement liée à l'effet du produit à caractère irritant.

Ainsi, les perturbations induites par un produit à caractère irritant sont suivies par une réponse plus ou moins intense de la peau visant à restaurer l'équilibre homéostatique rompu ou à réparer les dommages provoqués. Cette réponse peut être infraclinique, c'est à dire sans réaction inflammatoire évidente à l'oeil nu. Cependant, la réaction plus ou moins intense reste la réponse tissulaire la plus habituelle à l'aggression d'un produit irritant et la plus gênante pour l'utilisateur de ce produit à caractère irritant.

Lorsque le produit à caractère irritant atteint la peau, il peut réagir avec certaines substances préexistantes dans les cellules et les tissus et/ou libérer des substances intracellulaires. Ces substances libérées peuvent à leur tour devenir actives sur d'autres cibles dans l'épithélium ou le derme. Ainsi, s'amorce la cascade des réactions qui, par le recrutement de cellules sanguines et les substances qu'elles libèrent, donnent naissance au processus irritant qui se caractérise principalement par une irritation de la peau. Ce processus se traduit notamment à des degrés divers, dépendant principalement de la qualité et/ou de la quantité du produit apliqué et/ou de l'utilisateur de ce produit, par des sensations dysesthésiques (échauffement, sensations de brûlures, démangeaisons ou prurit, sensations de picotements, de tiraillements,...), par des rougeurs et/ou par un oedème.

Ces produits à caractère irritant peuvent être utilisés dans des compositions cosmétiques ou pharmaceutiques, et plus particulièrement dermatologiques, bien entendu pour d'autres effets. Ainsi, ils sont généralement utilisés en tant qu'agents actifs, tensioactifs, conservateurs, parfums, solvants ou propulseurs desdites compositions.
A cet égard on peut citer la demande de brevet GB-A-2263111 dans laquelle l'acide salicylique ou ses dérivés sont utilisés en association avec un analogue de l'arginine afin de ralentir l'effet létal de l'analogue de l'arginine lorsqu'il est injecté à un individu.

Mais du fait de leur caractère irritant, ces produits sont généralement utilisés en des doses très faibles. L'utilisation à faible quantité de ces produits peut alors s'avérer peu avantageuse par rapport à l'utilisation d'autres produits moins actifs, mais moins ou pas irritants et donc utilisés en plus grande quantité.

Par conséquent, il existe un besoin dans le domaine cosmétique et pharmaceutique de trouver un moyen permettant d'utiliser ces produits, sans que ces derniers présentent un caractère irritant reprochable par l'utilisateur.

Or, la Demanderesse a découvert que les inhibiteurs de NO-synthase permettent de limiter, voire de supprimer, le caractère irritant de ces produits.

Ainsi, la présente invention a pour objet l'utilisation d'une quantité efficace d'au moins un inhibiteur de NO-synthase pour la fabrication d'une composition pharmaceutique topique, la composition pharmaceutique étant destinée à diminuer l'effet irritant cutané de produits utilisés topiquement dans le domaine cosmétique ou pharmaceutique.

La composition cosmétique ou pharmaceutique topique comprenant l'inhibiteur de NO-synthase peut comprendre ou non le produit susceptible de provoquer une irritation cutanée.

Dans le cas où ces composés se trouvent dans la même composition, la présente invention concerne également une composition à usage topique, cosmétique ou pharmaceutique, caractérisée en ce qu'elle comprend, dans un milieu cosmétiquement ou pharmaceutiquement acceptable, une quantité efficace d'au moins un inhibiteur de NO-synthase et au moins un produit susceptible de provoquer une irritation cutanée.

La composition pharmaceutique est préférentiellement une composition dermatologique.

La présente invention. concerne aussi un procédé de traitement cosmétique, caractérisé en ce qu'il met en oeuvre la composition cosmétique selon l'invention.

La quantité efficace d'au moins un inhibiteur de NO-synthase selon l'invention est une quantité suffisante d'au moins un inhibiteur de NO-synthase pour que l'effet irritant cutané diminue, voire disparaisse. Ainsi, cette quantité est variable en fonction de la quantité et de la nature du produit à caractère irritant appliqué. Cependant, à titre d'illustration, une composition selon l'invention peut comprendre au moins un inhibiteur de NO-synthase à une concentration pondérale comprise entre 10⁻⁶ % et 10 % du poids total de la composition et préférentiellement entre 10⁻⁴ % et 1 % du poids total de la composition.

Dans la composition selon l'invention, la quantité du produit susceptible de provoquer une irritation cutanée peut donc correspondre à une quantité suffisante pour provoquer une irritation cutanée s'il était utilisé seul (sans l'inhibiteur de NO-synthase).

Dans la composition selon l'invention, la quantité du produit susceptible de provoquer une irritation cutanée peut donc correspondre à une quantité suffisante pour provoquer une irritation cutanée s'il était utilisé seul (sans l'inhibiteur de NO-synthase).

De nombreux produits appliqués topiquement présentent un caractère irritant, spécialement pour les personnes (utilisateurs) à peaux facilement irritables.

Ainsi, même les produits qui sont considérés comme inertes dans une composition cosmétique ou pharmaceutique, plus particulièrement dermatologique, peuvent présenter un caractère irritant lorsqu'ils sont appliqués sur la peau, le cuir chevelu, les ongles ou les muqueuses, tels que notamment des conservateurs, des tensio-actifs, des parfums, des solvants ou des propulseurs.

Aussi, des produits étant considérés comme des agents actifs dans des compositions cosmétiques ou pharmaceutiques peuvent présenter un caractère irritant lorsqu'ils sont appliqués sur la peau, le cuir chevelu, les ongles ou les muqueuses, on peut parler d'effet secondaire irritant, tels que notamment certains filtres solaires, les α-hydroxy-acides (glycolique, lactique, malique, citrique, tartrique, mandélique ), les β-hydroxy-acides (acide salicylique et ses dérivés), les α-céto-acides, les β-céto-acides, les rétinoïdes (rétinol et ses esters, rétinal, acide rétinoïque et ses dérivés, rétinoïdes, notamment ceux décrits dans les documents FR-A-2 570 377, EP-A-199 636, EP-A-325 540, EP-A-402 072), les anthralines (dioxyanthranol), les anthranoïdes (par exemple ceux décrits dans le document EP-A- 319028), les peroxydes (notamment le peroxyde de benzoyle), le minoxidil et ses dérivés, les sels de lithium, les antiprolifératifs, tels que le 5-fluorouracyle ou le méthotrexate, certaines vitamines, telles que la vitamine D et ses dérivés, la vitamine B9 et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les dépigmentants (hydroquinone), la capsaïcine, les actifs antipoux (pyréthrine), les agents détergents ioniques et non ioniques et des propigmentants (la dihydroxyacétone, les proralènes et les méthylangécilines).

Parmi ces produits à effet secondaire irritant, l'invention s'applique plus particulièrement aux rétinoïdes.

Parmi les rétinoïdes, on peut citer plus particulièrement l'acide rétinoïque tout-trans, l'acide rétinoïque 13-cis, l'acide carboxylique 2-(5,6,7,8-tetrahydro-5,5,8,8-tetraméthyl-2naphtyl)-6-benzo[b]thiophen, l'acide 6-[3-(1-adamanthyl)-4-méthoxyphényl]-2-naphtanoïque vendu sous le nom Adapalène ™ par la société Galderma, le Tazarotène ™ vendu par la société Allergan.

Parmi la vitamine D et ses dérivés, on peut citer plus particulièrement la vitamine D3, la vitamine D2, la 1, 25-diOH vitamine D3 (le calcitriol), le calcipotriol, la 1, 24-diOH vitamine D3 (tel que le tacalcitol), la 24, 25-diOH vitamine D3, la 1-OH vitamine D2, la 1, 24-diOH vitamine D2.

Parmi les dérivés de l'acide salicylique, on peut citer plus particulièrement l'acide n-octanoyl-5-salicylique et l'acide n-dodécanoyl-5-salicylique ou leurs esters.

Le monoxyde d'azote (NO) est enzymatiquement généré par la L-arginine, l'enzyme étant nommé la NO-synthase.

Les inhibiteurs de NO-synthase sont selon l'invention des produits qui permettent in situ sur l'homme d'inhiber partiellement, voire totalement, la synthèse de monoxyde d'azote (NO).

Cette enzyme existe sous deux formes, la forme constitutive et la forme inductible (Médecine/Sciences, 1992, 8, pp. 843-845). Parmi les inhibiteurs, on préfère utiliser les inhibiteurs de NO-synthase constitutive, c'est à dire les inhibiteurs qui inhibent autant ou plus la NO-synthase constitutive que la NO-synthase inductible. Les tests pour identifier les inhibiteurs de NO-synthase constitutive ou inductible sont notamment décrits dans le brevet US 5132453.

Parmi ces inhibiteurs de NO-synthase constitutive, on préfère les inhibiteurs de NO-synthase endothéliale.

En effet, il semble, sans vouloir se lier à une quelconque théorie de l'invention, que la diminution de l'irritation observée dans la présente invention est dûe principalement à l'inhibition des NO-synthases constitutives, et plus particulièrement à l'inhibition de la NO-synthase des cellules endothéliales.

Ainsi, parmi ces inhibiteurs de la NO-synthase constitutive, on peut citer plus particulièrement la N^{G}-monométhyl-L-arginine (NMMA), l'ester méthylé de la N^{G}-nitro-L-arginine (NAME), la N^{G}-nitro-L-arginine (NNA), la N^{G}-amino-L-arginine (NAA), la N^{G}.N^{G}-diméthyl-arginine (la diméthylarginine asymétrique, dénommée ADMA).

On utilise préférentiellement la NMMA, la NAME, la NNA et la ADMA.

Les inhibiteurs de la NO-synthase peuvent être utilisés seuls ou en mélange.

Les inhibiteurs de la NO-synthase peuvent être utilisés autant à titre préventif qu'à titre curatif.

La présente invention présente notamment l'avantage de pouvoir augmenter la quantité d'agents actifs à caractère irritant dans des compositions cosmétiques ou pharmaceutiques par rapport à la quantité normalement utilisée, en vue d'une efficacité de ces derniers améliorée. Ainsi, on peut utiliser les hydroxyacides jusqu'à 50 % du poids de la composition ou les rétinoïdes jusqu'à 5 %, sans aucune gène pour l'utilisateur.

Le ou les inhibiteurs de NO-synthase peuvent être utilisés par voie topique.

Par voie topique, on préfère l'application directe sur la peau, le cuir chevelu, les ongles ou les muqueuses.

Les compositions selon l'invention peuvent se présenter sous toutes les formes galéniques. Ces compositions sont préparées selon les méthodes usuelles.

Un milieu cosmétiquement ou dermatologiquement acceptable correspond généralement à un milieu compatible avec la peau, le cuir chevelu, les ongles ou les muqueuses. La composition comprenant l'inhibiteur de NO-synthase peut donc être appliquée sur le visage, le cou, les cheveux et les ongles, ou toute autre zone cutanée du corps (régions axillaires, sous-mammaires, plis du coude etc.).

Par voie topique, les compositions selon l'invention se présentent notamment sous forme de solutions aqueuses, hydroalcooliques ou huileuses, de dispersions du type lotion ou sérum, de gels anhydres ou lipophiles, d'émulsions de consistance liquide ou semi-liquide du type lait, obtenues par dispersion d'une phase grasse dans une phase aqueuse (H/E) ou inversement (E/H), ou de suspensions ou d'émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Elles peuvent être également utilisées pour le cuir chevelu sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des mousses à raser, des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires ou mieux après solaires, des lotions, gels ou mousses pour le soin de la peau, comme des lotions de nettoyage ou de désinfection, des lotions anti-solaires, des lotions de bronzage artificiel, des compositions pour le bain, des compositions désodorisantes contenant un agent bactéricide, des gels ou lotions après-rasage, des crèmes épilatoires, des compositions contre les piqûres d'insectes, des compositions anti-douleur ou des compositions pour traiter certaines maladies de la peau comme celles citées précédemment.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

Les inhibiteurs de NO-synthase peuvent être aussi incorporés dans diverses compositions pour soins ou traitements capillaires, et notamment des shampooings éventuellement antiparasitaires, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Les compositions de l'invention peuvent aussi être à usage bucco-dentaire, par exemple une pâte dentifrice ou un bain de bouche. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémuisionnants utilisés dans la composition sous forme d'émulsion sont choisis parmi ceux classiquement utilisés dans les domaines cosmétique et pharmaceutique. L'émulsionnant et le coémulsionnant sont présents, dans la composition, en une proportion allant de 0,3 % à 30 % en poids, et de préférence de 0,5 à 30 % ou mieux de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition de l'invention peut contenir également des adjuvants habituels dans le domaine cosmétique ou pharmaceutique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine cosmétique ou pharmaceutique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits végétaux, notamment ceux d'Aloe Vera.

Comme actifs lipophiles, on peut utiliser le rétinol (vitamine A) et ses dérivés, le tocophérol (vitamine E) et ses dérivés, les acides gras essentiels, les céramides, les huiles essentielles.

On peut entre autres associer les inhibiteurs de NO-synthase à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que notamment les rétinoïdes, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide citrique et de manière générale les acides de fruits et les bêta-hydroxyacides comme l'acide salicylique et ses dérivés notamment alcoylés comme, l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

Bien entendu, l'homme de l'art veille à choisir le ou les éventuels composés présents dans la composition selon l'invention de manière telle que les propriétés attachées intrinsèquement à la présente invention ne soient pas, ou substantiellement pas, altérées.

Les compositions pharmaceutiques selon l'invention conviennent particulièrement bien dans les domaines de traitement suivants, ces traitements étant particulièrement bien adaptés lorsque ces compositions comprennent des rétinoides :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,

La présente invention a en outre pour objet un procédé de traitement cosmétique, caractérisé par le fait qu'il met en oeuvre la composition cosmétique selon l'invention.

Préférentiellement, le procédé de traitement cosmétique consiste à appliquer sur la peau, sur le cuir chevelu, et/ou sur les muqueuses, une composition telle que décrite ci-dessus.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions après-solaires sur la peau ou sur les cheveux secs, application d'une lotion pour cheveux sur cheveux mouillés, de shampooings ou encore application de dentifrice sur les gencives.

Dans le domaine cosmétique, les compositions selon l'invention conviennent, en fonction des agents actifs contenus dans cette composition, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### EXEMPLE 1

Cet exemple a pour but de mettre en évidence l'activité anti-irritante orale *in vivo* de l'ester méthylé de la N^{G}-nitro-L-arginine utilisé en curatif.

Le test utilisé pour évaluer cette activité est celui de l'oedème de l'oreille de souris (souche Balb/C) induit par application topique de l'acide carboxylique 2-(5,6,7,8-tetrahydro-5,5,8,8-tetraméthyl-2naphtyl)-6-benzo[b]thiophen à 0,01% en poids. Selon ce modèle, la réponse à une application topique de l'acide carboxylique 2-(5,6,7,8-tetrahydro-5,5,8,8-tetraméthyl-2naphtyl)-6-benzo[b]thiophen sur l'oreille se traduit par une augmentation de l'épaisseur de l'oreille qui devient maximale au bout de 5 jours après l'application. Cette augmentation de l'épaisseur de l'oreille de souris semble être due à une augmentation de l'épaisseur de l'épiderme et à une apparition d'un oedème dermique. Cette réponse peut donc être facilement mesurée à l'aide d'un appareil, tel que l'oditest.

Le protocole opératoire exact est le suivant : 10 souris sont tout d'abord traitées avec le produit actif à caractère irritant, en procédant sur l'une de leur oreille à une application topique à un temps t=0 avec 20 µl d'une solution d'acétone comprenant 0,01% en poids d'acide carboxylique 2-(5,6,7,8-tetrahydro-5,5,8,8-tetraméthyl-2naphtyl)-6-benzo[b]thiophen. On fait absorber par voie orale à 5 (= groupe 2) des 10 souris ainsi traitées de l'ester méthylé de la N^{G}-nitro-L-arginine dans de l'eau de boisson à partir de t=0 et ceci une fois par jour pendant 11 jours (concentration ester méthylé de la N^{G}-nitro-L-arginine de 1mg/ml, soit 170 ± 40 mg/Kg par jour). Les 5 souris n'ayant pas absorbé l'ester méthylé de la N^{G}-nitro-L-arginine constituent le groupe 1. La réponse oedémateuse est quantifiée par une mesure de l'épaisseur de l'oreille. Les résultats sont ensuite exprimés en % d'augmentation de l'épaisseur de l'oreille de souris par rapport à l'augmentation de l'épaisseur observée sur l'autre oreille qui n'avait été, quant à elle, traitée (dans les mêmes conditions que ci-dessus) que par une solution d'acétone sans actif (oreille témoin ou de référence).

Les résultats obtenus sont les suivants :
Après 5 jours de traitement, l'augmentation de l'épaisseur de l'oreille de souris est à son maximum (100 %) pour le groupe 1 et est à 70 % pour le groupe 2.

Les résultats ci-dessus mettent clairement en évidence une inhibition de 30 % de l'oedème de l'oreille pour les souris traitées par cet inhibiteur de NO-synthase.

De plus, aucun signe de toxicité n'a été observé et l'évolution pondérale n'a pas été modifiée chez les souris traitées par cet inhibiteur.

### EXEMPLE 2

Cet exemple a pour but de mettre en évidence l'activité anti-irritante topique *in vivo* de la N^{G}N^{G}-diméthylarginine administrée en prévention.

Le test utilisé pour évaluer cette activité est le même que celui utilisé dans l'exemple 1.

Le protocole opératoire exact est le suivant : 5 souris sont tout d'abord traitées avec un gel comprenant comme seul agent actif de la N^{G}N^{G}-diméthylarginine à 1% en poids, en procédant sur l'une de leur oreille à une application topique par jour pendant 4 jours. On n'observe pas d'augmentation de l'épaisseur de l'oreille des souris ainsi traitées. Puis, on applique topiquement sur l'oreille de ces 5 souris traitées au préalable par la N^{G}N^{G}-diméthylarginine (groupe A) et sur l'oreille de 5 souris non traitées (groupe B), à un temps t=0, 20 µl d'une solution d'acétone comprenant de l'acide carboxylique 2-(5,6,7,8-tetrahydro-5,5,8,8-tetraméthyl-2naphtyl)-6-benzo[b]thiophen à 0,01% en poids. La réponse oedémateuse est quantifiée par une mesure de l'épaisseur de l'oreille. Les résultats sont ensuite exprimés en % d'augmentation de l'épaisseur de l'oreille de souris par rapport à l'augmentation de l'épaisseur observée sur l'autre oreille qui n'avait été, quant à elle, traitée (dans les mêmes conditions que ci-dessus) que par une solution d'acétone sans actif (oreille et oedème témoin ou de référence).

En comparant les groupes A et B, les résultats obtenus sont les suivants :
La N^{G}N^{G}-diméthylarginine appliquée topiquement une fois par jour pendant 4 jours avant l'application du produit à caractère irritant (l'acide carboxylique 2-(5,6,7,8-tetrahydro-5,5,8,8-tetraméthyl-2naphtyl)-6-benzo[b]thiophen) réduit de 24 % l'amplitude et de 50 % l'aire sous la courbe de la réponse induite par le produit à caractère irritant (la courbe correspondant à l'épaisseur de l'oreille en fonction des jours de lecture).

### EXEMPLE 3

Cet exemple a pour but de mettre en évidence l'activité anti-irritante topique *in vivo* de la N^{G}monométhyl-L-arginine (L-NMMA) utilisée à titre curatif.

Le test utilisé pour évaluer cette activité est le même que celui utilisé dans l'exemple 1.

Le protocole opératoire exact est le suivant : 10 souris sont tout d'abord traitées avec le produit actif à caractère irritant, en procédant sur l'une de leur oreille à une application topique à un temps t=0 avec 20 µl d'une solution d'acétone comprenant 0,01% en poids d'acide carboxylique 2-(5,6,7,8-tetrahydro-5,5,8,8-tetraméthyl-2naphtyl)-6-benzo[b]thiophen. On applique topiquement sur 5 (= groupe 2) des 10 souris ainsi traitées un gel comprenant la L-NMMA à 1% en poids 6 heures après l'application du produit à caractère irritant et ceci une fois par jour pendant 5 jours. Les 5 souris n'ayant pas été traitées par la L-NMMA constituent le groupe 1. La réponse oedémateuse est quantifiée par une mesure de l'épaisseur de l'oreille. Les résultats sont ensuite exprimés en % d'augmentation de l'épaisseur de l'oreille de souris par rapport à l'augmentation de l'épaisseur observée sur l'autre oreille qui n'avait été, quant à elle, traitée (dans les mêmes conditions que ci-dessus) que par une solution d'acétone sans actif (oreille témoin ou de référence).

Les résultats obtenus sont les suivants :
Après 5 jours de traitement, l'augmentation de l'épaisseur de l'oreille de souris est à son maximum (100 %) pour le groupe 1 et est à 72 % pour le groupe 2.
Les résultats mettent donc en évidence une inhibition de 28 % de l'oedème de l'oreille pour les souris traitées par cet inhibiteur de NO-synthase.

La L-NMMA réduit de 51 % l'aire sous la courbe de la réponse induite par le produit à caractère irritant (la courbe correspondant à l'épaisseur de l'oreille en fonction des jours de lecture).

Si l'on procède au même traitement en appliquant, à la place de la L-NMMA, de la bétaïne à 1 % ou à 5% ou de la N^{G}N^{G}-diméthyl-L-arginine à 1 % (la diméthyl-L-arginine symétrique, dénommée SDMA), on observe une inhibition respectivement de 9, de 16 et de 7% de l'oedème de l'oreille pour les souris traitées par ces produits qui ne sont pas des inhibiteurs de NO-synthase (voir notamment pour le SDMA : The Lancet, Vol.339 : 572-575). On observe également respectivement une réduction de 24, 13 et 27% de l'aire sous la courbe de la réponse induite par le produit à caractère irritant (la courbe correspondant à l'épaisseur de l'oreille en fonction des jours de lecture).

### EXEMPLE 4

On illustre ici des compositions conformes à l'invention se présentant sous la forme d'une lotion, d'un gel et d'une crème à usage topique.

| LOTION | |
|---|---|
| | % en poids |
| Disodium EDTA | 0,1 |
| Poloxamer 182 | 0,2 |
| Eau | q.s.p. 100 |
| Ethoxydiglycol | 5 |
| N^{G}N^{G}-diméthylarginine | 1 |

| GEL | |
|---|---|
| | % en poids |
| Disodium EDTA | 0,1 |
| Poloxamer 182 | 0,2 |
| Eau | q.s.p. 100 |
| Sepigel 305 vendu par Seppic | 3 |
| Ethoxydiglycol | 5 |
| N^{G}N^{G}-diméthylarginine | 1 |

| CREME | |
|---|---|
| | % en poids |
| Disodium EDTA | 0,1 |
| Poloxamer 182 | 0,2 |
| Eau | q.s.p. 100 |
| Conservateurs | 0,3 |
| Sepigel 305 vendu par Seppic | 3 |
| Huile de noyau d'abricot | 10 |
| Cyclométhicone | 5 |
| Ethoxydiglycol | 5 |
| Ester méthylé de | |
| la N^{G}-nitro-L-arginine | 1 |

| CREME | |
|---|---|
| émulsion huile dans eau | |
| | % en poids |
| N^{G}-monométhyl-L-arginine (NMMA) | 10⁻² |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,50 |
| Conservateur | 0,30 |
| Eau | qsp 100 |

| LOTION | |
|---|---|
| Adapalène^{™} | 0,010 g |
| N^{G}-monométhyl-L-arginine (NMMA) | 0,100g |
| Polyéthylène glycol (PEG 400) | 69,890 g |
| Ethanol à 95% | 30,000 g |

## Revendications

1. Utilisation d'une quantité efficace d'au moins un inhibiteur de NO-synthase pour la fabrication d'une composition pharmaceutique topique, destiné à diminuer l'effet irritant cutané de produits utilisés topiquement dans le domaine pharmaceutique.

2. Utilisation selon la revendication 1, **caractérisée en ce que** l'on utilise au moins un inhibiteur de NO-synthase à une concentration pondérale comprise entre 10⁻⁶ % et 10 % du poids total de la composition et préférentiellement entre 10⁻⁴ % et 1 % du poids total de la composition.

3. Utilisation selon l'une des revendications précédentes, **caractérisée en ce que** le produit à caractère irritant appliqué de manière topique sur la peau, le cuir chevelu, les ongles ou les muqueuses est un composé choisi parmi des conservateurs, des tensio-actifs, des parfums, des solvants ou des propulseurs.

4. Utilisation selon l'une des revendications précédentes, **caractérisée en ce que** le produit à caractère irritant appliqué de manière topique sur la peau, le cuir chevelu, les ongles ou les muqueuses est un composé choisi parmi certains filtres solaires, les α-hydroxy-acides, les β-hydroxy-acides, tels que l'acide salicylique et ses dérivés, les α-céto-acides, les β-céto-acides, les rétinoïdes, les anthralines, les anthranoïdes, les peroxydes, le minoxidil et ses dérivés, les sels de lithium, les antiprolifératifs, la vitamine D et ses dérivés, la vitamine B9 et ses dérivés, les teintures ou colorants capillaires, la capsaïcine, les solutions alcooliques parfumantes, les agents antitranspirants, les actifs dépilatoires ou de permanentes, les dépigmentants, les actifs antipoux, les détergents et les propigmentants.

5. Utilisation selon la revendication précédente, **caractérisée en ce que** le produit à caractère irritant est choisi parmi les rétinoïdes.

6. Utilisation selon la revendication précédente, **caractérisée en ce que** les rétinoïdes sont choisies parmi l'acide rétinoïque tout-trans, l'acide rétinoïque 13-cis, l'acide carboxylique 2-(5,6,7,8-tetrahydro-5,5,8,8-tetraméthyl-2naphtyl)-6-benzo[b]thiophen, l'acide 6-[3-(1-adamanthyl)-4-méthoxyphényl]-2-naphtanoïque, le Tazarotène ^{™}.

7. Utilisation selon la revendication 4, **caractérisée en ce que** la vitamine D et ses dérivés sont choisis parmi la vitamine D3, la vitamine D2, la 1, 25-diOH vitamine D3 (le calcitriol), le calcipotriol, la 1, 24-diOH vitamine D3 (tel que le tacalcitol), la 24, 25-diOH vitamine D3, la 1-OH vitamine D2, la 1, 24-diOH vitamine D2.

8. Utilisation selon la revendication 4, **caractérisée en ce que** les dérivés de l'acide salicylique sont choisis parmi l'acide n-octanoyl-5-salicylique et l'acide n-dodécanoyl-5-salicylique ou leurs esters.

9. Utilisation selon l'une quelconque des revendications précédentes, **caractérisée en ce que** les inhibiteurs de la NO-synthase sont les inhibiteurs de NO-synthase constitutive.

10. Utilisation selon la revendication précédente, **caractérisée en ce que** les inhibiteurs de NO-synthase constitutive sont les inhibiteurs de NO-synthase endothéliale.

11. Utilisation selon l'une des revendications 9 ou 10, **caractérisée en ce que** les inhibiteurs de la NO-synthase sont choisis parmi la N^{G}-monométhyl-L-arginine (NMMA), l'ester méthylé de la N^{G}-nitro-L-arginine (NAME), la N^{G}-nitro-L-arginine (NNA), la N^{G}-amino-L-arginine (NAA), la N^{G}.N^{G}-diméthyl-arginine (la diméthylarginine asymétrique, dénommée ADMA).

12. Utilisation selon la revendication précédente, **caractérisée en ce que** les inhibiteurs de la NO-synthase sont choisis parmi l'ester méthyle de la N^{G}-nitro-L-arginine, la N^{G}.N^{G}-diméthyl-arginine, la N^{G}-nitro-L-arginine et la N^{G}-monométhyl-L-arginine (NMMA).

13. Utilisation selon l'une quelconque des revendications précédentes, **caractérisée en ce que** les inhibiteurs de la NO-synthase sont utilisés seuls ou en mélange.

14. Composition à usage topique, cosmétique ou pharmaceutique, **caractérisée en ce qu'**elle comprend, dans un milieu cosmétiquement ou pharmaceutiquement acceptable, une quantité efficace d'au moins un inhibiteur de NO-synthase et au moins un produit susceptible de provoquer une irritation cutanée choisi parmi des conservateurs, des tensio-actifs, des parfums, des solvants, des propulseurs, des certains filtres solaires, les α-hydroxy-acides, les α-céto-acides, les β-céto-acides, les rétinoïdes, les anthralines, les anthranoïdes, les peroxydes, le minoxidil et ses dérivés, les sels de lithium, les antiprolifératifs, la vitamine D et ses dérivés, la vitamine B9 et ses dérivés, les teintures ou colorants capillaires, la capsaïcine, les solutions alcooliques parfumantes, les agents antitranspirants, les actifs dépilatoires ou de permanentes, les dépigmentants, les actifs antipoux, les détergents et les propigmentants.

15. Composition selon la revendication précédente, **caractérisée en ce que** le produit susceptible de provoquer une irritation cutanée est choisi parmi les rétinoides.

16. Composition selon la revendication précédente, **caractérisée en ce que** les rétinoïdes sont choisies parmi l'acide rétinoïque tout-trans, l'acide rétinoïque 13-cis, l'acide carboxylique 2-(5,6,7,8-tetrahydro-5,5,8,8-tetraméthyl-2naphtyl)-6-benzo[b]thiophen, l'acide 6-[3-(1-adamanthyl)-4-méthoxyphényl]-2-naphtanoïque, le Tazarotène ™.

17. Composition selon la revendication 14, **caractérisée en ce que** la vitamine D et ses dérivés sont choisis parmi la vitamine D3, la vitamine D2, la 1, 25-diOH vitamine D3 (le calcitriol), le calcipotriol, la 1, 24-diOH vitamine D3, tel que le tacalcitol, la 24, 25-diOH vitamine D3, la 1-OH vitamine D2, la 1, 24-diOH vitamine D2.

18. Composition selon l'une des revendications 14 à 17, **caractérisée en ce que** les inhibiteurs de la NO-synthase sont sont les inhibiteurs de NO-synthase constitutive.

19. Composition selon la revendication précédente, **caractérisée en ce que** les inhibiteurs de NO-synthase constitutive sont les inhibiteurs de NO-synthase endothéliale.

20. Composition selon l'une des revendications 18 ou 19, **caractérisée en ce que** les inhibiteurs de la NO-synthase sont choisis parmi la N^{G}-monométhyl-L-arginine (NMMA), l'ester méthylé de la N^{G}-nitro-L-arginine (NAME), la N^{G}-nitro-L-arginine (NNA), la N^{G}-amino-L-arginine (NAA), la N^{G}.N^{G}-diméthyl-arginine (la diméthylarginine asymétrique, dénommée ADMA).

21. Composition selon la revendication précédente, **caractérisée en ce que** les inhibiteurs de la NO-synthase sont choisis parmi l'ester méthyle de la N^{G}-nitro-L-arginine, la N^{G}.N^{G}-diméthyl-arginine, la N^{G}-nitro-L-arginine et la N^{G}-monométhyl-L-arginine (NMMA).

22. Composition selon l'une quelconque des revendications 14 à 21, **caractérisée en ce que** les inhibiteurs de la NO-synthase sont utilisés seuls ou en mélange.

23. Composition selon l'une quelconque des revendications 14 à 22, **caractérisée en ce qu'**elle comprend au moins un inhibiteur de NO-synthase à une concentration pondérale comprise entre 10⁻⁶ % et 10 % du poids total de la composition et préférentiellement entre 10⁻⁴ % et 1 % du poids total de la composition.

24. Composition selon l'une quelconque des revendications 14 à 23, **caractérisée en ce qu'**elle est formulée de façon à être appliquée sur la peau, sur le cuir chevelu et/ou sur les muqueuses.

25. Composition selon l'une quelconque des revendications 14 à 24, **caractérisée en ce que** la composition pharmaceutique est une composition dermatologique.

26. Procédé de traitement cosmétique, **caractérisé en ce qu'**il met en oeuvre la composition cosmétique selon l'une des revendications 14 à 24.

## Claims

1. Use of an effective quantity of at least one NO-synthase inhibitor for the manufacture of a topical pharmaceutical composition, intended to reduce the cutaneous irritant effect of products topically used in the pharmaceutical field.

2. Use according to Claim 1, **characterized in that** at least one NO-synthase inhibitor is used at a concentration by weight of between 10⁻⁶% and 10% of the total weight of the composition and preferably between 10⁻⁴% and 1% of the total weight of the composition.

3. Use according to either of the preceding claims, **characterized in that** the product having an irritant character applied topically to the skin, the scalp, the nails or the mucous membranes is a compound chosen from preservatives, surfactants, perfumes, solvents or propellants.

4. Use according to one of the preceding claims, **characterized in that** the product having an irritant character applied topically to the skin, the scalp, the nails or the mucous membranes is a compound chosen from some sunscreens, α-hydroxy acids, β-hydroxy acids, such as salicylic acid and its derivatives, α-keto acids, β-keto acids, retinoids, anthralins, anthranoids, peroxides, minoxidil and its derivatives, lithium salts, antiproliferative agents, vitamin D and its derivatives, vitamin B9 and its derivatives, hair dyes or colorants, capsaicin, perfuming alcoholic solutions, antiperspirants, depilatory or permanent waving active agents, depigmenting agents, antilouse active agents, detergents and propigmenting agents.

5. Use according to the preceding claim, **characterized in that** the product having an irritant character is chosen from retinoids.

6. Use according to the preceding claim, **characterized in that** the retinoids are chosen from all-trans-retinoic acid, 13-cis-retinoic acid, 2-(5,6,7,8-tetrahydro-5,5,8,8-tetramethyl-2-naphthyl)-6-benzo[b]thiophenecarboxylic acid, 6-[3-(1-adamantyl)-4-methoxyphenyl]-2-naphthanoic acid, Tazarotene^{™}_{.}

7. Use according to Claim 4, **characterized in that** the vitamin D and its derivatives are chosen from vitamin D₃, vitamin D₂, 1,25-dish vitamin D₃ (calcitriol), calcipotriol, 1,24-diOH vitamin D₃ (such as tacalcitol), 24,25-diOH vitamin D₃, 1-OH vitamin D₂, 1,24-diOH vitamin D₂.

8. Use according to Claim 4, **characterized in that** the salicylic acid derivatives are chosen from 5-n-octanoylsalicylic acid and 5-n-dodecanoylsalicylic acid or their esters.

9. Use according to any one of the preceding claims, **characterized in that** the NO-synthase inhibitors are inhibitors of constitutive NO-synthase.

10. Use according to the preceding claim, **characterized in that** the inhibitors of constitutive NO-synthase are inhibitors of endothelial NO-synthase.

11. Use according to either of Claims 9 and 10, **characterized in that** the NO-synthase inhibitors are chosen from N^{G}-monomethyl-L-arginine (NMMA), the methyl ester of N^{G}-nitro-L-arginine (NAME), N^{G}-nitro-L-arginine (NNA), N^{G}-amino-L-arginine (NAA), N^{G},N^{G}-dimethylarginine (asymmetric dimethylarginine, called ADMA).

12. Use according to the preceding claim, **characterized in that** the NO-synthase inhibitors are chosen from the methyl ester of N^{G}-nitro-L-arginine, N^{G},N^{G}-dimethylarginine, N^{G}-nitro-L-arginine and N^{G}-monomethyl-L-arginine (NMMA).

13. Use according to any one of the preceding claims, **characterized in that** the NO-synthase inhibitors are used alone or as a mixture.

14. Cosmetic or pharmaceutical composition for topical use, **characterized in that** it comprises, in a cosmetically or pharmaceutically acceptable medium, an effective quantity of at least one NO-synthase inhibitor and at least one product capable of causing skin irritation chosen from preservatives, surfactants, perfumes, solvents, propellants, some sunscreens, α-hydroxy acids, α-keto acids, β-keto acids, retinoids, anthralins, anthranoids, peroxides, minoxidil and its derivatives, lithium salts, antiproliferative agents, vitamin D and its derivatives, vitamin B9 and its derivatives, hair dyes or colorants, capsaicin, perfuming alcoholic solutions, antiperspirants, depilatory or permanent waving active agents, depigmenting agents, antilouse active agents, detergents and propigmenting agents.

15. Composition according to the preceding claim, **characterized in that** the product capable of causing skin irritation is chosen from retinoids.

16. Composition according to the preceding claim, **characterized in that** the retinoids are chosen from all-trans-retinoic acid, 13-cis-retinoic acid, 2-(5,6,7,8-tetrahydro-5,5,8,8-tetramethyl-2-naphthyl)-6-benzo[b]thiophenecarboxylic acid, 6-[3-(1-adamantyl)-4-methoxyphenyl]-2-naphthanoic acid, Tazarotene^{™}_{.}

17. Composition according to Claim 14, **characterized in that** the vitamin D and its derivatives are chosen from vitamin D₃, vitamin D₂, 1,25-diOH vitamin D₃ (calcitriol), calcipotriol, 1,24-diOH vitamin D₃ such as tacalcitol, 24,25-diOH vitamin D₃, 1-OH vitamin D₂, 1,24-diOH vitamin D₂.

18. Composition according to one of Claims 14 to 17, **characterized in that** the NO-synthase inhibitors are inhibitors of constitutive NO-synthase.

19. Composition according to the preceding claim, **characterized in that** the inhibitors of constitutive NO-synthase are inhibitors of endothelial NO-synthase.

20. Composition according to either of Claims 18 and 19, **characterized in that** the NO-synthase inhibitors are chosen from N^{G}-monomethyl-L-arginine (NMMA), the methyl ester of N^{G}-nitro-L-arginine (NAME), N^{G}-nitro-L-arginine (NNA), N^{G}-amino-L-arginine (NAA), N^{G},N^{G}-dimethylarginine (asymmetric dimethylarginine, called ADMA).

21. Composition according to the preceding claim, **characterized in that** the NO-synthase inhibitors are chosen from the methyl ester of N^{G}-nitro-L-arginine, N^{G},N^{G}-dimethylarginine, N^{G}-nitro-L-arginine and N^{G}-monomethyl-L-arginine (NMMA).

22. Composition according to any one of Claims 14 to 21, **characterized in that** the NO-synthase inhibitors are used alone or as a mixture.

23. Composition according to any one of Claims 14 to 22, **characterized in that** it comprises at least one NO-synthase inhibitor at a concentration by weight of between 10⁻⁶% and 10% of the total weight of the composition and preferably between 10⁻⁴% and 1% of the total weight of the composition.

24. Composition according to any one of Claims 14 to 23, **characterized in that** it is formulated so as to be applied to the skin, the scalp and/or the mucous membranes.

25. Composition according to any one of Claims 14 to 24, **characterized in that** the pharmaceutical composition is a dermatological composition.

26. Process of cosmetic treatment, **characterized in that** it uses the cosmetic composition according to one of Claims 14 to 24.

## Patentansprüche

1. Verwendung einer wirksamen Menge mindestens eines Inhibitors der NO-Synthase zur Herstellung einer pharmazeutischen topischen Zusammensetzung, der die irritierende Wirkung von in der Pharmazie topisch angewendeten Produkten auf die Haut vermindern soll.

2. Verwendung nach Anspruch 1, **dadurch gekennzeichnet, dass** mindestens ein Inhibitor der NO-Synthase in einer Gewichtskonzentration von 10⁻⁶ bis 10 % des Gesamtgewichts der Zusammensetzung und vorzugsweise von 10⁻⁴ bis 1 % des Gesamtgewichts der Zusammensetzung verwendet wird.

3. Verwendung nach einem der vorhergehenden Ansprache, **dadurch gekennzeichnet, dass** es sich bei dem Produkt mit irritierender Wirkung, das auf topischem Wege auf die Haut, die Kopfhaut, die Nägel oder die Schleimhäute aufgebracht wird, um eine Verbindung handelt, die unter Konservierungsstoffen, grenzflächenaktiven Stoffen, Parfums, Lösemitteln oder Treibmitteln ausgewählt ist.

4. Verwendung nach einem der vorhergehenden Ansprüche, **dadurch gekennzeichnet, dass** es sich bei dem Produkt mit irritierender Wirkung, das auf topischem Wege auf die Haut, die Kopfhaut, die Nägel oder die Schleimhäute aufgebracht wird, um eine Verbindung handelt, die unter bestimmten Sonnenschutzfiltern, α-Hydroxysäuren, β-Hydroxysäuren, wie Salicylsäure und ihren Derivaten, α-Ketosäuren, β-Ketosäuren, Retinoiden, Anthralinen, Anthranoiden, Peroxiden, Minoxidil und seinen Derivaten, Lithiumsalzen, Cytostatika, Vitamin D und seinen Derivaten, Vitamin B9 und seinen Derivaten, Färbemitteln oder Farbstoffen für das Haar, Capsaicin, parfümierenden alkoholischen Lösungen, Antitranspirantien, Haarentfernungsmitteln oder Mitteln für die permanente Verformung, depigmentierenden Wirkstoffen, Wirkstoffen gegen Läuse, reinigenden Mitteln und pigmentierungsfördernden Wirkstoffen ausgewählt ist.

5. Verwendung nach dem vorhergehenden Anspruch, **dadurch gekennzeichnet, dass** das Produkt mit irritierender Wirkung unter den Retinoiden ausgewählt ist.

6. Verwendung nach dem vorhergehenden Anspruch, **dadurch gekennzeichnet, dass** die Retinoide unter *all*-*trans*-Retinsâure, 13-*cis*-Retinsäure, 2-(5,6,7,8-Tetrahydro-5,5,8,8-tetramethyl-2-naphthyl)-6-benzo[b]thiophen-carbonsäure, 6-[3-(1-Adamantyl)-4-methoxyphenyl]-2-naphthoesäure und Tazaroten^{™} ausgewählt sind.

7. Verwendung nach Anspruch 4, **dadurch gekennzeichnet; dass** das Vitamin D und seine Derivate unter Vitamin D3, Vitamin D2, 1,25-Dihydroxyvitamin D3 (Calcitriol), Calcipotriol, 1,24-Dihydroxyvitamin D3 (wie Tacalcitol), 24,25-Dihydroxyvitamin D3, 1-Hydroxyvitamin D2 und 1,24-Dihydroxyvitamin D2 ausgewählt sind.

8. Verwendung nach Anspruch 4, **dadurch gekennzeichnet, dass** die Salicylsäurederivate unter 5-(n-Octanoyl)-salicylsäure, 5-(n-Dodecanoyl)-salicylsäure oder den Estern dieser Verbindungen ausgewählt sind.

9. Verwendung nach einem der vorhergehenden Ansprache, **dadurch gekennzeichnet, dass** es sich bei den Inhibitoren der NO-Synthase um Inhibitoren der konstitutiven NO-Synthase handelt.

10. Verwendung nach dem vorhergehenden Anspruch, **dadurch gekennzeichnet, dass** es sich bei den Inhibitoren der konstitutiven NO-Synthase um Inhibitoren der endothelialen NO-Synthase handelt.

11. Verwendung nach einem der Ansprüche 9 oder 10, **dadurch gekennzeichnet, dass** die Inhibitoren der NO-Synthase unter N^{G}-Monomethyl-L-arginin (NMMA), dem Methylester von N^{G}-Nitro-L-arginin (NAME), N^{G}-Nitro-L-arginin (NNA), N^{G}-Amino-L-arginin (NAA) und N^{G},N^{G}-Dimethylarginin (asymmetrisches Dimethylarginin, das als ADMA bezeichnet wird) ausgewählt sind.

12. Verwendung nach dem vorhergehenden Anspruch, **dadurch gekennzeichnet, dass** die Inhibitoren der NO-Synthase unter dem Methylester von N^{G}-Nitro-L-arginin, N^{G},N^{G}-Dimethylarginin, N^{G}-Nitro-L-arginin und N^{G}-Monomethyl-L-arginin (NMMA) ausgewählt sind.

13. Verwendung nach einem der vorhergehenden Ansprüche, **dadurch gekennzeichnet, dass** die Inhibitoren der NO-Synthase allein oder im Gemisch verwendet werden.

14. Zusammensetzung zur topischen Anwendung, wobei es sich um eine kosmetische oder pharmazeutische Zusammensetzung handelt, **dadurch gekennzeichnet, dass** sie in einem kosmetisch oder pharmazeutisch akzeptablen Medium eine wirksame Menge mindestens eines Inhibitors der NO-Synthase und mindestens ein Produkt enthält, das eine Hautirritation hervorrufen kann und das unter Konservierungsstoffen, grenzflächenaktiven Stoffen, Parfums, Lösemitteln, Treibmitteln, bestimmten Sonnenschutzfiltern, α-Hydroxysäuren, β-Hydroxysäuren, α-Ketosäuren, β-Ketosäuren, Retinoiden, Anthralinen, Anthranoiden, Peroxiden, Minoxidil und seinen Derivaten, Lithiumsalzen, Cytostatika, Vitamin D und seinen Derivaten, Vitamin B9 und seinen Derivaten, Färbemitteln oder Farbstoffen für das Haar, Capsaicin, parfümierenden alkoholischen Lösungen, Antitranspirantien, Haarentfernungsmitteln oder Mitteln für die dauerhafte Verformung, depigmentierenden Wirkstoffen, Wirkstoffen gegen Läuse, reinigenden Mitteln und pigmentierungsfördernden Wirkstoffen ausgewählt ist.

15. Zusammensetzung nach dem vorhergehenden Anspruch, **dadurch gekennzeichnet, dass** das Produkt, das eine Hautirritation hervorrufen kann, unter den Retinoiden ausgewählt ist.

16. Zusammensetzung nach dem vorhergehenden Anspruch, **dadurch gekennzeichnet, dass** die Retinoide unter *all*-*trans*-Retinsäure, 13-*cis*-Retinsäure, 2-(5,6,7,8-Tetrahydro-5,5,8,8-tetramethyl-2-naphthyl)-6-benzo[b]thiophen-carbonsäure, 6-[3-(1-Adamantyl)-4-methoxyphenyl]-2-naphthoesäure und Tazaroten^{™} ausgewählt sind.

17. Zusammensetzung nach Anspruch 14, **dadurch gekennzeichnet, dass** das Vitamin D und seine Derivate unter Vitamin D3, Vitamin D2, 1,25-Dihydroxyvitamin D3 (Calcitriol), Calcipotriol, 1,24-Dihydroxyvitamin D3, wie Tacalcitol, 24,25-Dihydroxyvitamin D3, 1-Hydroxyvitamin D2 und 1,24-Dihydroxyvitamin D2 ausgewählt sind.

18. Zusammensetzung nach einem der Ansprüche 4 bis 7, **dadurch gekennzeichnet, dass** es sich bei den Inhibitoren der NO-Synthase um Inhibitoren der konstitutiven NO-Synthase handelt.

19. Zusammensetzung nach dem vorhergehenden Anspruch, **dadurch gekennzeichnet, dass** es sich bei den Inhibitoren der konstitutiven NO-Synthase um Inhibitoren der endothelialen NO-Synthase handelt.

20. Zusammensetzung nach einem der Ansprüche 18 oder 19, **dadurch gekennzeichnet, dass** die Inhibitoren der NO-Synthase unter N^{G}-Monomethyl-L-arginin (NMMA), dem Methylester von N^{G}-Nitro-L-arginin (NAME), N^{G}-Nitro-L-arginin (NNA), N^{G}-Amino-L-arginin (NAA) und N^{G},N^{G}-Dimethylarginin (asymmetrisches Dimethylarginin, das als ADMA bezeichnet wird) ausgewählt sind.

21. Zusammensetzung nach dem vorhergehenden Anspruch, **dadurch gekennzeichnet, dass** die Inhibitoren der NO-Synthase unter dem Methylester von N^{G}-Nitro-L-arginin, N^{G},N^{G}-Dimethylarginin, N^{G}-Nitro-L-arginin und N^{G}-Monomethyl-L-arginin (NMMA) ausgewählt sind.

22. Zusammensetzung nach einem der Ansprüche 14 bis 21, **dadurch gekennzeichnet, dass** die Inhibitoren der NO-Synthase allein oder im Gemisch verwendet werden.

23. Zusammensetzung nach einem der Ansprüche 14 bis 22 , **dadurch gekennzeichnet, dass** sie mindestens einen Inhibitor der NO-Synthase in einer Gewichtskonzentration von 10⁻⁶ bis 10 % des Gesamtgewichts der Zusammensetzung und vorzugsweise von 10⁻⁴ bis 1 % des Gesamtgewichts der Zusammensetzung enthält.

24. Zusammensetzung nach einem der Ansprüche 14 bis 23, **dadurch gekennzeichnet, dass** sie so formuliert ist, dass sie auf die Haut, die Kopfhaut und/ oder die Schleimhäute aufgebracht werden kann.

25. Zusammensetzung nach einem der Ansprüche 14 bis 24, **dadurch gekennzeichnet, dass** die pharmazeutische Zusammensetzung eine dermatologische Zusammensetzung ist.

26. Verfahren zur kosmetischen Behandlung, **dadurch gekennzeichnet, dass** die kosmetische Zusammensetzung nach einem der Ansprüche 14 bis 24 verwendet wird.
